# EUROPÄISCHE PATENTSCHRIFT

(11) **EP 1 372 579 B1**
(45) Veröffentlichungstag und Bekanntmachung des Hinweises auf die Patenterteilung: **07.12.2005**
(21) Anmeldenummer: 02722285.0
(22) Anmeldetag: 30.03.2002
(51) Int. Cl.: A61K 7/06, A61K 7/50

(54) **REINIGUNGSTÜCHER ZUR HAARPFLEGE**
CLEANING TOWELS FOR HAIR CARE
SERVIETTES DE NETTOYAGE POUR SOINS CAPILLAIRES

(30) Priorität: 07.04.2001 DE 10117500
(43) Veröffentlichungstag der Anmeldung: 02.01.2004
(73) Patentinhaber: Cognis IP Management GmbH, 40589 Düsseldorf (DE)
(72) Erfinder: SEIPEL, Werner, 40723 Hilden (DE); HENSEN, Hermann, 42781 Haan (DE); GOEBELS, Dagmar, 46562 Voerde (DE)
(86) Internationale Anmeldenummer: PCT/EP2002/003561
(87) Internationale Veröffentlichungsnummer: WO 2002/080865

(56) Entgegenhaltungen:
- WO-A-99/55303
- DE-A- 4 204 185

## Beschreibung

### Gebiet der Erfindung

Die Erfindung befindet sich auf dem Gebiet der Haarpflege und betrifft die Verwendung von Reinigungstüchern, die mit einer speziellen Tensidlösung imprägniert sind.

### Stand der Technik

Reinigungstücher sind eine Anwendungsform, die sich zunehmender Bedeutung in der Kosmetik erfreut. Neben üblichen Haushaltstüchern, Toilettenpapieren, Papiertaschentüchern, Gesichtsreinigungstüchern, Abschminktüchern, Erfrischurigstüchern und dergleichen sind sogenannte "wet wipes" bereits in vielen Variationen kommerziell erhältlich. Es handelt sich dabei um Feuchttücher, die textile Gewebe oder aber auch Tissuepapiere darstellen, welche mit einer Reinigungslösung getränkt sind.
Zahlreiche Patentanmeldungen beschreiben Reinigungstücher mit desinfizierender Wirkung, stellvertretend sei die **WO 95/17175** oder **WO 98/55096** genannt
Als Trägermaterial zur Imprägnierung mit Reinigungslösungen werden unterschiedliche Gewebe beschrieben (**WO 99/13861** und **WO 01/08657**). In der Patentanmeldung **WO 99/66793** werden Wirkstoffe genannt, die auf Tücher aufgetragen werden können.
Neben der Reinigung wurde zunehmend auch die Pflege der Haut berückslchtigt. So werden beispielsweise in der internationalen Patentanmeldung **WO 95/35411** Feuchttücher vorgeschlagen, welche mit einer Lotion imprägniert sind, die neben Mineralöl, Fettsäureester, Fettalkoholethoxylate und Fettalkohole enthalten.
Vornehmlich werden diese Feuchttücher für die Anwendung auf der Haut eingesetzt.
Auch, wenn die Anwendung im Haarbereich erwähnt ist, so lösen die genannten Anmeldungen nicht das Problem des Verklebens der Haare oder des Verbleibs der Komponenten auf der Kopfhaut, welches eine sehr gute Verträglichkeit der Verbindungen voraussetzt.

Eine erste Aufgabe der vorliegenden Erfindung hat somit darin bestanden, Reinigungstücher für die Haarpflege unter Verwendung spezieller gut verträglicher Tenside zur Verfügung zu stellen, welche eine einfache, saubere und schnelle Verwendung ermöglichen und einen guten Reinigungseffekt zeigen. Diese Tenside sollen nicht zu einem Verkleben der Haare führen, denn es soll dabei auch möglich sein, dass die Formulierungen nach der Anwendung in den Haaren verbleiben.
Ein weiteres Problem tritt bei der Herstellung der Feuchttücher auf. Um das Gewebe oder Tissuepapier mit der Reinigungslösung zu tränken, wird es entweder mit ihr besprüht oder in sie eingetaucht, wobei es in beiden Fällen durch Schaumbildung oder eine zu geringe Benetzung zu einer Verminderung des Durchsatzes in der Produktion kommen kann. Eine weitere Aufgabe der Erfindung hat somit darin bestanden, eine Tensidlösung zur Verfügung zu stellen, die durch ihre Viskosität und Schaumarmut rasch In die Tücher einzieht, so dass die Tenside gut im Gewebe verteilt werden können und somit eine technisch einfache und daher kostengünstige Herstellung von Feuchttüchem gestattet. Sollen die Reinigungstücher jedoch in trockener Form vorliegen und vor der Anwendung mit Wasser befeuchtet werden, so ist während der Herstellung ein schnelles Verdampfen des Lösungsmittels erwünscht.

### Beschreibung der Erfindung

Gegenstand der Erfindung ist die Verwendung von Reinigungstüchern, die sich dadurch auszeichnen, dass sie mit einer Tensidlösung imprägniert sind, enthaltend
(a) Alk(en)yloligoglycoside
(b) Alkoholpolyglycolethern in Kombination mit und/oder
(c) Hydroxymischethern
zur Pflege und Reinigung von Haaren

Es wurde gefunden, dass Reinigungstücher, die Tensidmischungen von Alk(en)yloligoglykosiden in Kombination mit Alkoholpolyglycolether oder Hydroxymischethern enthalten, die komplexe Aufgabe in ausgezeichneter Weise erfüllen. Sie zeigen nach der Anwendung an Haaren einen deutlichen Reinigungseffekt ohne ein klebriges Gefühl zu hinterlassen. Dabei kann durch die Auswahl des Lösungsmittels zusätzlich die Reinigungsleistung, das Volumen des Haares und der Glanz beeinflußt werden. Als "Leave on" ist die aufgetragene Tensidmischung sehr gut verträglich, so dass ein nachträgliches Spülen der Haare nicht notwendig war. Eine einfache und hygienische Anwendung zur Reinigung der Haare ist somit auch dort möglich, wo ein herkömmlicher Waschprozeß nicht durchgeführt werden kann, wie beispielsweise bei bettlägrigen Patienten oder während der Reise.

Zusätzlich erweisen sich die aufgetragenen Tensidlösungen auf Basis von Alk(en)yloligoglycosiden und Alkoholpolyglycolethern und/oder Hydroxymischethern in der Herstellung als gut verarbeitbar, niedrigviskos und praktisch schaumfrei, so dass sie schnell vom entsprechenden Gewebe aufgenommen wurden. Dieser Effekt wird noch verstärkt durch den Einsatz eines alkoholisch/wässrigen Lösungsmittels.

### Alkyl- und/oder Alkenyloligoglykoside

Bei Alk(en)yloligoglycosiden handelt es sich um bekannte nichtionische Tenside dar, die der Formel **(I)** folgen,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schriftum sei hier auf die Schriften **EP-A1 0301298** und **WO 90/03977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-%-C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3. Die Alkyl- und/oder Alkenyloligoglykoside können - bezogen auf die feuchten Tücher - in Mengen von 0,05 bis 2 und vorzugsweise 0,5 bis 1 Gew.-% und - bezogen auf die Konzentrate in Mengen von 30 bis 80, vorzugsweise 50 bis 70 Gew.-% eingesetzt werden, wobei das Gewichtsverhältnis Alkoholpolyglycolether zu Glykosid im Bereich von 10 : 90 bis 90 : 10, vorzugsweise 25 : 75 bis 75 : 25 und insbesondere 40 : 60 bis 60 : 40 liegen kann.

### Alkoholpolyglycolether

Alkoholpolyglycolether stellen bekannte nichtionische Tenside dar, welche üblicherweise erhalten werden, indem man Ethylenoxid und/oder Propylenoxid, blockweise oder in random-Verteilung an geeignete primäre Alkohole oder Polyole addiert. Üblicherweise folgen die Polyglycolether der Formel **(II),** 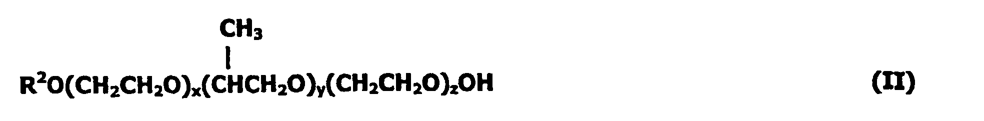 in der R² für einen linearen und/oder verzweigten Alkyl- und/oder Alkenylrest mit 6 bis 22, vorzugsweise 8 bis 18 und insbesondere 10 bis 12 Kohienstotfiatomen, einen Ethylenglycol- oder Glycerinrest, x und z unabhängig von einander für 0 oder Zahlen von 1 bis 40 und y für Zahlen von 1 bis 40 steht; die Polyglycolether enthalten also zwingend mindestens eine Propylenoxid-Einheit. Typische Beispiele sind die Anlagerungsprodukte von durchschnittlich 1 bis 40, vorzugsweise 5 bis 30 und insbesondere 8 bis 15 Mol Ethylenoxid und/oder 1 bis 10, vorzugsweise 2 bis 5 Mol Propylenoxid an Fettalkohole, Oxoalkohole oder Alfole, wie etwa Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen sowie Ethylenglycol oder Glycerin.

### Hydroxymischether

Hydroxymischether (HME) stellen bekannte nichtionische Tenside mit unsymmetrischer Etherstruktur und Polyalkylenglycolanteilen dar, welche man beispielsweise erhält, indem man Olefinepoxide mit Fettalkoholpolyglycolethern einer Ringöffnungsreaktion unterwirft. Typischerweise folgen die Hydroxymischether der allgemeinen Formel **(III)**, 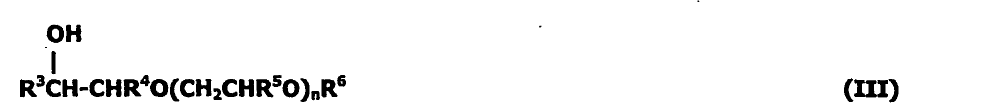 in der R³ für einen linearen oder verzweigten Alkylrest mit 2 bis 18, vorzugsweise 10 bis 16 Kohlenstoffatomen, R⁴ für Wasserstoff oder einen linearen oder verzweigten Alkylrest mit 2 bis 18 Kohlenstoffatomen, R⁵ für Wasserstoff oder Methyl, R⁶ für einen linearen oder verzweigten, Alkyl- und/oder Alkenylrest mit 1 bis 22, vorzugsweise 8 bis 18 Kohlenstoffatomen und n für Zahlen von 1 bis 50, vorzugsweise 2 bis 25 und insbesondere 5 bis 15 steht, mit der Maßgabe, daß die Summe der Kohlenstoffatome in den Resten R³ und R⁴ mindestens 4, vorzugsweise 6 bis 18 und insbesondere 8 bis 12 beträgt. Wie aus der Formel hervorgeht, können die HME Ringöffnungsprodukte sowohl von innenständigen Olefinen (R⁴ ungleich Wasserstoff) oder endständigen Olefinen (R⁴ gleich Wasserstoff) sein, wobei letztere im Hinblick auf die leichtere Herstellung und die vorteilhafteren anwendungstechnischen Eigenschaften bevorzugt sind. Gleichfalls kann der polare Teil des Moleküls eine Polyethylen- oder eine Polypropylenkette sein; ebenfalls geeignet sind gemischte Ketten von PE- und PP-Einheiten, sei es in statistischer oder Blockverteilung. Typische Beispiele sind Ringöffnungsprodukte von 1,2-Hexenepoxid, 2,3-Hexenepoxid, 1,2-Octenepoxid, 2,3-Ocetenepoxid, 3,4-Octenepoxid, 1,2-Decenepoxid, 2,3-Decenepoxid, 3,4-Decenepoxid, 4,5-Decenepoxid, 1,2-Dodecenepoxid, 2,3-Dodecenepoxid, 3,4-Dodecenepoxid, 4,5-Dodecenepoxid, 5,6-Dodecenepoxid, 1,2-Tetradecenepoxid, 2,3-Tetradecenepoxid, 3,4-Tetradecenepoxid, 4,5-Tetradecenepoxid, 5,6-Tetradecenepoxid, 6,7-Tetradecenepoxid, 1,2-Hexadecenepoxid, 2,3-Hexadecenepoxid, 3,4-Hexadecenepoxid, 4,5-Hexadecenepoxid, 5,6-Hexadecenepoxid, 6,7-Hexadecenepoxid, 7,8-Hexadecenepoxid, 1,2-Octadecenepoxid, 2,3-Octadecenepoxid, 3,4-Octadecenepoxid, 4,5-Octadecenepoxid, 5,6-Octadecenepoxid, 6,7-Octadecenepoxid, 7,8-Octadecenepoxid und 8,9-Octadecenepoxid sowie deren Gemische mit Anlagerungsprodukten von durchschnittlich 1 bis 50, vorzugsweise 2 bis 25 und insbesondere 5 bis 15 Mol Ethylenoxid und/oder 1 bis 10, vorzugsweise 2 bis 8 und insbesondere 3 bis 5 Mol Propylenoxid an gesättigte und/oder ungesättigte primäre Alkohole mit 6 bis 22, vorzugsweise 12 bis 18 Kohlenstoffatomen, wie z.B. Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearytalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen.

### Tissuepapiere und Tissuegewebe für Reinigungstücher

Tissuepapiere und -gewebe, auf die sich die vorliegende Erfindung bezieht, können ein- oder mehrlagig aufgebaut sein. In der Regel weisen die Papiere ein Quadratmetergewicht von 10 bis 65, vorzugsweise 15 bis 30 g und eine Dichte von 0,6 g/cm³ und weniger auf.
Neben den papierbasierten Tissues kommen auch entsprechende Tissuegewebe in Frage, die aus Faser- oder Fleecestoff hergestellt werden. Zu Beipielen für Naturfasern zählen Seide, Cellulose, Keratin, Wolle, Baumwolle, Jute, Leinen, Flaks, für synthetische Fasern Acetat-, Acrylat-, Celluloseester-, Polyamid-, Polyester-, Polyolefin-, Polyvinylalkohol-, Polyurethan- Fasern. Bevorzugt ist hier die Anwendung von hydrophilen Fasern, Naturfasern wie Baumwollgewebe und Baumwollmischgewebe, besonders bevorzugt sind durch Additive hydrophilierte Polyolefingewebe. Umsetzungsprodukte von 1 Teil Polyethylenglykol mit 2 Teilen Fettsäuren mit 10 bis 12 C-Atomen oder deren Derivaten werden hierbei zur Hydrophilierung der Polyolefinhaltigen Gewebe eingesetzt.
Das Gewebe kann dabei in Handschuhform vorliegen und ist dann bevorzugt mehrlagig, so dass die innere Gewebelage des Handschuhs eine Barrierefunktion hat und einen Schutz vor der Berührung der Hand mit der Formulierung oder mit Feuchtigkeit bietet.

### Reinigungslösung und Lösungsmittel

Das Gewichtsverhältnis vom trockenen Gewebe zu aufgebrachter Reinigungslösung soll 1:0.1 bis 1 : 4, vorzugsweise 1:0,5 bis 1:3 und besonders bevorzugt 1:1 bis 1:2 betragen.
Dabei soll das Lösungsmittel der Reinigungslösung aus Wasser oder bevorzugt Wasser/Alkoholgemischen bestehen. Als Alkohole werden Isopropanol, Propanol und Ethanol eingesetzt. Die für die Imprägnierung eingesetzten Reinigungslösungen enthalten 0 bis 95 Gew.% Alkohol, vorzugsweise 3 bis 70 Gew.% Alkohol, besonders bevorzugt 5 bis 20 Gew.% Alkohol. Die Solubilisierung des Haarfettes wird bei der Anwendung feuchter Reinigungstücher nicht nur durch die Art und Menge der eingesetzten Tenside, sondern auch durch den Alkoholgehalt beeinflußt, somit läßt sich die Reinigungswirkung, das Volumen und der Glanz des gereinigten Haares durch den Gehalt an Alkohol steuern.
Auch bei der Herstellung trockener Reinigungstücher, die vor der Anwendung angefeuchtet werden, hat der Alkoholgehalt der verwendeten Lösung einen entscheidenden Einfluß, da die Trocknungsrate und damit die Herstellungskosten optimiert werden können.

### Herstellungsverfahren

Das Verfahren zur Herstellung von Reinigungstüchern ist **dadurch gekennzeichnet, dass** man ein Gewebe mit einer Tensidlösung, enthaltend
(a) Alk(en)yloligoglycoside
(b) Alkoholpolyglycolether und/oder
(c) Hydroxymischether
befeuchtet und gegebenenfalls nachfolgend das Lösungsmittel bis auf einen Restgehalt von 0.1 bis 3 Gew.%, vorzugsweise von unter 0.1 Gew. % - bezogen auf das Gewicht des Reinigungstuches heraustrocknet.
Dabei kann das Befeuchten durch Aufsprühen der Tensidlösung auf das Gewebe oder Eintauchen der Gewebe In die Tensidlösung durchgeführt werden. Ist ein nachträgliches Abdampfen des Lösungsmittels gewünscht, so werden die befeuchteten Tücher einer erhöhten Temperatur, einem warmen Luftstrom oder einer Vakuumtrocknung ausgesetzt.

### Gewerbliche Anwendbarkeit

Vorgeschlagen wird die Verwendung von Reinigungstüchern, **dadurch gekennzeichnet, dass** sie mit einer Tensidlösung imprägniert sind, die typischerweise folgende Zusammensetzung hat:
(a) 0,1 - 10 Gew.%, vorzugsweise 0,5 - 5% Gew.%, insbesondere 1 - 3 Gew.% Alk(en)yloligoglycoside
(b) 0,01 - 5 Gew.% vorzugsweise 0,1 - 3 Gew.%, insbesondere 0,3 - 0,5 Gew.% Alkoholpolyglycolether und/oder
(c) 0,01 - 5 Gew.% vorzugsweise 0,1 - 3 Gew.%; insbesondere 0,3 - 0,5 Gew.% Hydroxymischether
zur Pflege und Reinigung von Haaren.

Die zur Imprägnierung eingesetzten Tensidlösungen können ferner als weitere Hilfs- und Zusatzstoffe milde Tenside, Emulgatoren, Konsistenzgeber, Verdickungsmittel, Stabilisatoren, Polymere, Siliconverbindungen, Lecithine, Phospholipide, biogene Wirkstoffe, UV-Lichtschutzfaktoren, Antioxidantien, Antischuppenmittel, Filmbildner, Hydrotrope, Solubilisatoren, Konservierungsmittel, Parfümöle, Farbstoffe und dergleichen enthalten.

### Weitere Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside, Alkylphenolpolyglycolether, Fettsäurepolyglycolester, Fettsäureamidpolyglycolether, Fettaminpolyglycolether, alkoxylierte Triglyceride, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Fettsäureglucamide, Alkylamidobetaine, Amphoacetale und/oder Proteinfettsäurekondensate, letztere vorzugsweise auf Basis von Weizenproteinen.

### Emulgatoren

Als weitere Emulgatoren kommen beispielsweise nichtionogene Tenside aus mindestens einer der folgenden Gruppen in Frage:
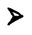 Anlagerungsprodukte von 2 bis 30 Mol Ethylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
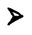 Anlagerungsprodukte von 1 bis 15 Mol Ethylenoxid an Ricinusöl und/oder gehärtetes Ricinusöl;
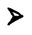 Anlagerungsprodukte von 15 bis 60 Mol Ethylenoxid an Ricinusöl und/oder gehärtetes Ricinusöl;
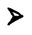 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
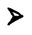 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3-bis 18 Kohienstoßatomen sowie deren Addukte mit 1 bis 30 Mol Ethylenoxid;
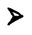 Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin.
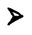 Mono-, Di- und Trialkylphosphate sowie Mono-, Di- und/oder Tri-PEG-alkylphosphate und deren Salze;
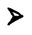 Wollwachsalkohole;
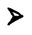 Polysiloxan-Polyalkyl-Polyether-Copolymere bzw. entsprechende Derivate;
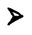 Block-Copolymere z.B. Polyethylenglycol-30 Dipolyhydroxystearate;
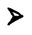 Polymeremulgatoren, z.B. Pemulen-Typen (TR-1,TR-2) von Goodrich;
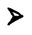 Polyalkylenglycole sowie
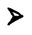 Glycerincarbonat.

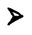 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

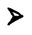 Sorbitanester

Als Sorbitanester kommen Sorbitanmonoisostearat, Sorbitansesqiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

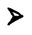 Polyglycerinester

Typische Beispiele für geeignete Polyglycerinester sind Polyglyceryl-2 Dipolyhydroxystearate (Dehymuls® PGPH), Polyglycerin-3-Diisosteate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

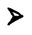 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

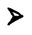 Amphothere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Hemologenvertellung oder Alkyloligoglucoside sowie Elektrolyte wie Kochsalz und Ammoniumchlorid.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vemetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil.** **108,** **95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil.** **91,** **27 (1976).**

### UV-Uchtschutzfilter und Antioxidantien

Unter UV-Lichtschutrfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
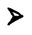 3-Benzylidencampher bzw. 3-Benzylidennorcampher und dessen Derivate, z.B. 3-(4-Methylbenzyliden)campher wie in der **EP 0693471 B1** beschrieben;
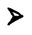 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
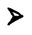 Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
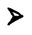 Ester der Salicylsäure, vorzugsweise Salicylsäure-2-ethylhexylester, Salicylsäure-4-iso-propylbenzylester, Salicylsäurehomomenthylester;
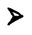 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
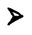 Ester der Benzalmalonsäure, vorzugsweise 4-Methoxybenzmalonsäuredi-2-ethylhexyl-ester;
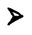 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
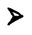 Propan-1,3-dione, wie z.B. 1-(4-tert.Butylphenyl)-3-(4'-methoxyphenyl)propan-1,3-dion;
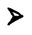 Ketotricyclo(5.2.1.0)decan-Derivate, wie in der **EP 0694521 B1** beschrieben.

Als wasserlösliche Substanzen kommen in Frage:
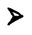 2-Phenylbenzimidazol-5-sulfonsäure und deren Alkali-, Erdalkali-, Ammonium-, Alkylammonium-, Alkanolammonium- und Glucammoniumsalze;
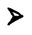 Sulfonsäurederivate von Benzophenonen, vorzugsweise 2-Hydroxy-4-methoxybenzophenon-5-sulfonsäure und ihre Salze;
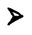 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Flter kommen insbesondere Derivate des Benzoylmethans in Frage, wie beispielsweise 1-(4'-tert.Butyiphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den vorgenannten Lichtschutzstoffen können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren. (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. α-Carotin, β-Carotin, Lycopin)-und deren- Derivate, Chlorogensäure-und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fett säuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol,-Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Filmbildner

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinomonoethanolaminsalz), Baypival® (Climbazole), Ketoconazol®, (4-Acetyl-1-{-4-[2-(2,4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
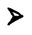 Glycerin;
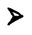 Alkylenglycole, wie beispielsweise Ethylenglycol, Diethylenglycol, Propylenglycol, Butylenglycol, Hexylenglycol sowie Polyethylenglycole mit einem durchschnitdichen Molekulargewicht von 100 bis 1.000 Dalton;
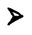 technische-Oligoglyceringemische mit einem Eigenkondensationsgrad von 1,5 bis 10 wie etwa technische Diglyceringemische mit einem Diglyceringehalt von 40 bis 50 Gew.-%;
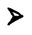 Methyolverbindungen, wie insbesondere Trimethylolethan, Trimethylolpropan, Trimethylolbutan, Pentaerythrit und Dipentaerythrit;
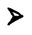 Niedrigalkylglucoside, insbesondere solche mit 1 bis 8 Kohlenstoffen im Alkylrest, wie beispielsweise Methyl- und Butylglucosid;
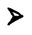 Zuckeralkohole mit 5 bis 12 Kohlenstoffatomen, wie beispielsweise Sorbit oder Mannit,
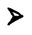 Zucker mit 5 bis 12 Kohlenstoffatomen, wie beispielsweise Glucose oder Saccharose;
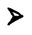 Aminozucker, wie beispielsweise Glucamin;
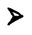 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadein und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen.

### Beispiele

Verschiedene Imprägnierlösungen wurden durch einfaches Vermischen der Komponenten hergestellt; anschheßend wurden dünne Baumwollhandschuhe von 12 g Gewicht mit je 20 g der Tensidlösungen 3 oder 4 befeuchtet und Haare - in Form kurzer oder schulterlanger Haarlänge - damit eingerieben.
Die behandelten Haare wiesen durch ein vermehrtes Volumen, einen geringeren Fettgehalt und einen angenehmen Glanz einen deutlichen Reinigungseffekt auf.
In einem zweiten Versuch wurden die mit der Tensidlösung 3 und 4 imprägnierten Handschuhe bei 30°C über 24 Stunden getrocknet. Die trockenen imprägnierten Handschuhe wurden vor dem Gebrauch erneut mit je 18 g Wasser befeuchtet. Die auf diese Weise behandelten Haare zeigten den gleichen Effekt, wenn auch in etwas geringerem Maße.

**Tabelle 1**

| **Zusammensetzung der Imprägnierlösungskonzentrate Mengenangaben als Gew.-%** | | |
|---|---|---|
| **Zusammensetzung** | **1** | **2** |
| C₁₂-C₁₄-Kokosalkohol+5EO+4PO | 10,0 | - |
| Dehypon Ke 3447 (HME) ¹⁾ | - | 11,1 |
| C₈-C₁₀ Alkyloligoglucosid | 63,0 | 63,0 |
| Bronidox ²⁾ | 0,3 | 0,3 |
| Citronensäure | 2,5 | 2,5 |
| Wasser | Ad 100,0 | ad 100,0 |

| | | |
|---|---|---|
| 1) C8-C10-Fettalkohol Ethylenoxid/Propylenoxid 22/1 + Decenepoxid | | |
| 2) 2) Propylene Glycol (and) 5-Bromo-5-Nitro-1,3-Dioxane | | |

**Tabelle 2**

| **Zusammensetzung der Imprägnierlösungen Mengenangaben als Gew.-%** | | |
|---|---|---|
| **Zusammensetzung** | **3** | **4** |
| Tensidlösungskonzentrat 1 | 4,0 | |
| Tensidlösungskonzentrat 2 | - | 3,8 |
| Parfümöl | 0,2 | 0,2 |
| Konservierungsmittel | 0,1 | 0,1 |
| Ethanol | 8,0 | 8,2 |
| Wasser | ad 100,0 | ad 100,0 |
| | | |
| PH-Wert | 5,5 | 5,5 |

## Patentansprüche

1. Verwendung von Reinigungstüchern, **dadurch gekennzeichnet, dass** sie mit einer Tensidlösung imprägniert sind, enthaltend
(a) Alk(en)yloligoglycoside
(b) Alkoholpolyglycolethern in Kombination mit und/oder
(c) Hydroxymischethern
zur Pflege und Reinigung von Haaren.

2. Verwendung von Reinigungstüchern nach Anspruch 1, **dadurch gekennzeichnet, dass** das Gewichtsverhältnis trockenes Gewebe zu aufgebrachter Reinigungslösung 1:0.1 bis 1 : 4 beträgt.

3. Verwendung von Reinigungstüchern nach den Ansprüchen 1 und/oder 2,. **dadurch gekennzeichnet, dass** die Reinigungstücher Alk(en)yloligoglykoside in Mengen von 0,05 bis 2 Gew.-% - bezogen auf das feuchte Reinigungstuch - enthalten.

4. Verwendung von Reinigungstüchern nach mindestens einem der Ansprüche 1 bis 3, **dadurch gekennzeichnet, dass** die Reinigungstücher Alkoholpolyglycolether und/oder Hydroxymischether in Mengen von 0,01 bis 1 Gew.-% - bezogen auf das feuchte Reinigungstuch - enthalten.

5. Verwendung von Reinigungstüchern nach mindestens einem der Ansprüche 1 bis 4, **dadurch gekennzeichnet, dass** die Reinigungstücher Wasser in Mengen von 10 bis 70 Gew.-% - bezogen auf das feuchte Reinigungstuch - enthalten.

6. Verwendung von Reinigungstüchern nach mindestens einem der Ansprüche 1 bis 5, **dadurch gekennzeichnet, dass** die Reinigungstücher Alkohol in Mengen von 10 bis 70 Gew.-% - bezogen auf das feuchte Reinigungstuch - enthalten.

7. Verwendung von Reinigungstüchern nach mindestens einem der Ansprüche 1 bis 6, , **dadurch gekennzeichnet, dass** die Reinigungstücher weitere übliche Hilfs- und Zusatzstoffe enthalten.

8. Verfahren zur Herstellung von Reinigungstüchern nach mindestens einem der Ansprüche 1 bis 7 **dadurch gekennzeichnet, dass** man ein Gewebe mit einer Tensidlösung, enthaltend
(a) Alk(en)yloligoglycoside
(b) Alkoholpolyglycolethern in Kombination mit und/oder
(c) Hydroxymischethern
befeuchtet und gegebenenfalls nachfolgend das Lösungsmittel bis auf einen Restgehalt von 0.1 bis 3 Gew.% - bezogen auf das Gewicht des Reinigungstuches heraustrocknet.

9. Verwendung von Reinigungstüchern nach mindestens einem der Ansprüche 1 bis 9, **dadurch gekennzeichnet, dass** sie vor der Anwendung mit Wasser befeuchtet werden.

10. Verwendung von Reinigungstüchern nach mindestens einem der Ansprüche 1 bis 10, **dadurch gekennzeichnet, dass** sie in Handschuhform vorliegen.

## Claims

1. The use of cleaning cloths, **characterized in that** they are impregnated with a surfactant solution containing
(a) alk(en)yl oligoglycosides in combination with
(b) alcohol polyglycol ethers and/or
(c) hydroxy mixed ethers
for the care and cleaning of hair.

2. The use of cleaning cloths as claimed in claim 1, **characterized in that** the ratio by weight of dry cloth to applied cleaning solution is 1:0.1 to 1:4.

3. The use of cleaning cloths as claimed in claims 1 and/or 2, **characterized in that** the cleaning cloths contain alk(en)yl oligoglycosides in quantities of 0.05 to 2% by weight, based on the wet cleaning cloth.

4. The use of cleaning cloths as claimed in at least one of claims 1 to 3, **characterized in that** the cleaning cloths contain alkyl polyglycol ethers and/or hydroxy mixed ethers in quantities of 0.01 to 1% by weight, based on the wet cleaning cloth.

5. The use of cleaning cloths as claimed in at least one of claims 1 to 4, **characterized in that** the cleaning cloths contain water in quantities of 10 to 70% by weight, based on the wet cleaning cloth.

6. The use of cleaning cloths as claimed in at least one of claims 1 to 5, **characterized in that** the cleaning cloths contain alcohol in quantities of 10 to 70% by weight, based on the wet cleaning cloth.

7. The use of cleaning cloths as claimed in at least one of claims 1 to 6, **characterized in that** the cleaning cloths contain other typical auxiliaries and additives.

8. A process for the production of the cleaning cloths claimed in at least one of claims 1 to 7, **characterized in that** a cloth is wetted with a surfactant solution containing
(a) alk(en)yl oligoglycosides in combination with
(b) alcohol polyglycolethers and/or
(c) hydroxy mixed ethers
and the solvent is then optionally removed by drying to a residual content of 0.1 to 3% by weight, based on the weight of the cleaning cloth.

9. The use of the cleaning cloths as claimed in at least one of claims 1 to 9, **characterized in that** they are wetted with water before use.

10. The use of the cleaning cloths as claimed in at least one of claims 1 to 10, **characterized in that** they are present in the form of gloves.

## Revendications

1. Utilisation d'étoffes de nettoyage,
**caractérisée en ce qu'**
elles sont imprégnées d'une solution tensioactive contenant :
a) des alc(én)yloligoglycosides en combinaison avec
b) des polyglycoléthers d'alcools et/ou
c) des hydroxyéthers mixtes,
pour l'entretien et le nettoyage des cheveux.

2. Utilisation d'étoffes de nettoyage selon la revendication 1,
**caractérisée en ce que**
le rapport en poids du tissu sec à la solution de nettoyage appliquée sur celui-ci est de 1 / 0, 1 à 1 / 4.

3. Utilisation d'étoffes de nettoyage selon la (les) revendication(s) 1 et/ou 2,
**caractérisée en ce que**
des étoffes de nettoyage contiennent des alc(én)ylologoglycosides dans des proportions de 0,05 à 2 % en poids, rapporté à l'étoffe de nettoyage humide.

4. Utilisation d'étoffes de nettoyage selon au moins une des revendications 1 à 3,
**caractérisée en ce que**
des étoffes de nettoyage contiennent des polyglycoléthers d'alcools et/ou des hydroxyéthers mixtes dans des proportions de 0,01 à 1 % en poids, rapporté à l'étoffe de nettoyage humide.

5. Utilisation d'étoffes de nettoyage selon au moins une des revendications 1 à 4,
**caractérisée en ce que**
des étoffes de nettoyage contiennent de l'eau dans des proportions de 10 à 70 % en poids, rapporté à l'étoffe de nettoyage humide.

6. Utilisation d'étoffes de nettoyage selon au moins une des revendications 1 à 5,
**caractérisée en ce que**
des étoffes de nettoyage contiennent un alcool dans des proportions de 10 à 70 % en poids, rapporté à l'étoffe de nettoyage humide.

7. Utilisation d'étoffes de nettoyage selon au moins une des revendications 1 à 6,
**caractérisée en ce que**
des étoffes de nettoyage contiennent d'autres adjuvants et additifs courants.

8. Procédé d'obtention d'étoffes de nettoyage selon au moins une des revendications 1 à 7,
**caractérisé en ce qu'**
on humidifie un tissu avec une solution tensioactive contenant :
a) des alc(én)yloligoglycosides en combinaison avec
b) des polyglycoléthers d'alcools et/ou
c) des hydroxyéthers mixtes,
puis on élimine ensuite éventuellement le solvant par séchage jusqu'à une teneur résiduelle de 0,1 à 3 % en poids, rapporté au poids de l'étoffe de nettoyage.

9. Utilisation d'étoffes de nettoyage selon au moins une des revendications 1 à 9,
**caractérisée en ce qu'**
elles sont humidifiées avec de l'eau avant utilisation.

10. Utilisation d'étoffes de nettoyage selon au moins une des revendications 1 à 10,
**caractérisée en ce qu'**
elles se présentent sous forme de gants.
